# EUROPEAN PATENT SPECIFICATION

(11) **EP 4 394 725 B1**
(45) Date of publication and mention of the grant of the patent: **18.11.2026**
(21) Application number: 22383293.2
(22) Date of filing: 27.12.2022
(51) Int. Cl.: G06V 10/764, A61B 5/00, G06V 20/52, G06V 40/10, A61B 5/11

(54) **METHOD AND SYSTEM FOR MONITORING BEDRIDDEN AND BED-RESTING PERSONS**
VERFAHREN UND SYSTEM ZUR ÜBERWACHUNG VON BETTLÄGERIGEN UND BETTRUHENDEN PERSONEN
PROCÉDURE ET SYSTÈME DE SURVEILLANCE DES PERSONNES ALITÉES ET DES PERSONNES SE REPOSANT DANS LEUR LIT

(43) Date of publication of application: 03.07.2024
(73) Proprietor: Fundacion Centro de Tecnologias de Interaccion Visual y comunicaciones Vicomtech, 20009 San Sebastian (ES)
(72) Inventor: Unzueta Irurtia, Luis, Donostia/San Sebastián (ES); Goenetxea Imaz, Jon, Donostia/San Sebastián (ES); Iriarte Satrustegi, Francisco Javier, Donostia/San Sebastián (ES); García Castaño, Jorge, Donostia/San Sebastián (ES); Elordi Hidalgo, Unai, Donostia/San Sebastián (ES); Otaegui Madurga, Oihana, Donostia/San Sebastián (ES)
(74) Representative: Balder IP Law, S.L.

(56) References cited:
- US-A1- 2012 075 464
- US-B1- 11 282 367

## Description

### TECHNICAL FIELD

This invention is related to the field of monitoring, surveillance and caring of bedridden and bed-resting persons, for example elderly people, disabled people and convalescent people. More particularly, the invention refers to methods and systems for monitoring bedridden and bed-resting people, and their body pose, that use computer vision algorithms.

### BACKGROUND OF THE INVENTION

Caring for a bedridden person with reduced mobility requires caregivers changing the person's in-bed body posture every few hours to help keep blood flowing. This helps the skin stay healthy and prevents bedsores. Turning a patient is a good time to check the skin for redness and sores. Other bedridden people with higher mobility may attempt to get out the bed (e.g., to go to the bathroom), but they might fall to the floor or not go back to bed. When there are many people to take care of, with different kind of needs, especially at night, an automated monitoring system to aid caregivers could be very helpful.

There are computer vision methods to detect people and body keypoints on images that can be used in many kinds of contexts. For example, the document "Face detection techniques: a review. Ashu Kumar et al. Artif. Intell. Rev. 52(2): 927-948 (2019)" discloses methods for face detection in digital images (human face detection methods). The document "A Review of Facial Landmark Extraction in 2D Images and Videos Using Deep Learning. Matteo Bodini. Big Data Cogn. Comput. 3(1): 14 (2019)" disclosed methods for facial landmark extraction on 2D images and video (human face landmarks detection methods). The document "Human detection techniques for real time surveillance: a comprehensive survey. MA Ansari et al. Multim. Tools Appl. 80(6): 8759-8808 (2021)" discloses methods for human detection and their classification based on various underlying factors (human body detection methods). The document "Human pose estimation and its application to action recognition: A survey. J. Vis. L. Song et al. Commun. Image Represent. 76: 103055 (2021)" discloses methods for human pose estimation and their application to action recognition (human body keypoints detection methods).

However, when these methods are used to monitor bedridden people, they are not able to characterize the pose of the persons in bed due to the huge occlusions caused by blankets and sheets that may be covering, at least partially, the body and/or face of the person. There is no perfect human body pose detection method for all kind of images obtained from bedridden people. Any detector using the aforementioned methods will provide false positive results and negative detection rates impossible to fully remove.

Thus, there is a need in the art for methods and systems able to monitor the body posture of bedridden and bed-resting people with a high degree of accuracy and reliability so that they can be remotely surveilled by their relatives or health staff when they are bedridden or temporarily convalescents (bed-resting) in their own houses or in residential homes, hospitals or in any other health facility.

Another relevant prior art document is the patent application US11282367B1.

### DESCRIPTION OF THE INVENTION

For overcoming the aforementioned drawbacks, a method and system for monitoring bedridden and bed-resting people, and their body pose, are herein provided. This solution exploits the advantages of state-of-the-art human face, facial landmarks, body, and body keypoints detection methods to effectively monitor bedridden and bed-resting people for, preferably, person-care applications. This solution can be also used for surveillance applications of healthy people or of persons immobilized in beds, e.g., in psychiatric institutions or prisons. More specifically, the solution herein disclosed combines all these detection methods to robustly detect the following events: whether the person is in the bed and the in-bed body pose (e.g., frontal, left, right), whether the person is attempting to get out the bed, whether the person has left the room where the bed is located, whether the person has received a visit, or whether the person has fallen to the floor.

A first object of the invention is a computer-implemented method for monitoring bedridden and bed-resting persons. The method herein disclosed is suitable for bedridden and bed-resting people although it may also be used for any kind of persons that should be monitored when they are in bed. The method comprises the steps of:
i. receiving, at a computing system, at least one top view image of a bed. This top view image may be preferably captured or generated by an imaging device, such as cameras or sensors, located in the ceiling of the room where the bed is placed and in a position that is centered relative to the bed. In some other embodiments, the imaging device may be located closer to the area of the bed where the pillow is located to have a better vision over the pillow area. As used herein the "top view image of the bed" refers to an image taken from above that contains the whole bed and eventually part of the area surrounding the bed. For example, the imaging device may be configured to capture an image of the bed and part of the bedroom where the bed is located. The images may be gathered by a monitoring system installed within the bedroom where the bed is located and may include cameras, video cameras, time of flight cameras, depth sensors, or any other imaging device capable of directly gathering images or generating them from the information that the imaging device is able to capture. As used herein, a "computing device" may be a desktop computer, laptop (or notebook) computer, workstation, tablet computer, mobile phone, smart device, switch, router, server, blade enclosure, or any other processing device or equipment including a processing resource. In examples described herein, a processing resource may include, for example, one processor or multiple processors included in a single computing device or distributed across multiple computing devices;
ii. delimiting, by the computing system, on the at least one image a first area corresponding to the pillow area and a second area corresponding to the rest of the bed. In other words, the computing system draws a first polygonal line (generally a rectangle) forming a first box that contains the portion of the bed where the pillow is placed, delimiting the first area, and a second polygonal line (generally another rectangle) forming a second box that contains the rest of the bed (excluding the portion to the bed where the pillow is placed), deliming the second area. Alternatively, this delimiting step could be manually carried out provided that the imaging devices that capture or generate the images and the beds being monitored are usually at fixed locations. In such case, these first and second boxes are provided to the computing system to delimit said first and second areas;
iii. applying, by the computing system, a face detection algorithm and a body detection algorithm on the at least one image to generate respective face bounding boxes and body bounding boxes on the at least one image. Each face bounding box contains a detectable portion of the face of a person in the image and each body bounding box contains a detectable portion of the body of the persons in the image. In fact, the face bounding boxes delimit the area in the at least one image that contains the detectable portion of the detected faces. Similarly, the body bounding boxes delimit the area in the at least one image that contains the detectable portion of the detected bodies. The face detection algorithms and the body detection algorithms used could be any of those algorithms well-known in the state of the art, such as those cited in the background of this document. As used herein, the detectable portions of the face and of the body may refer to those portions of the face and body of the person that the algorithm is able to detect because they are visible or not being visible because the algorithm is able to estimate their presence, location and contour in the image.
iv. applying, by the computing system and when the face detection algorithm has generated at least one face bounding box, i.e., the face detection algorithm has detected a face of a person within the at least one image, a face landmarks detection algorithm to detect key landmark points inside the corresponding at least one face bounding box. Depending on the face landmarks detection algorithm used the number and nature of the key landmark points may vary. Preferably, these face landmarks detection algorithms will detect at least the eyes, nose and mouth within the at least one face bounding box. The face landmarks detection algorithms used may be any of those algorithms well-known in the state of the art, such as those cited in the background of this document
v. checking, by the computing system and when at least one face bounding box has been generated (i.e., when at least a detectable portion of the face of a user has been detected), a position of the detected key landmark points and of corners of the at least one face bounding box relative to the first area and the second area;
vi. checking, by the computing system and when at least one body box has been generated (i.e., when at least a detectable portion of the body of a user has been detected), the position of the at least one body bounding box relative to the first area;
vii. determining, by the computing system, that there is a bedridden or bed-resting person in the at least one image when:
   at least one of the key landmark points is located within the first or second area.

In other words, when the face of the person is located within any of the two areas that jointly delimit the area of the bed;
and/or
at least one point belonging to the generated at least one body bounding box is located within the first area, or when at least one point belonging to the first area is located within the generated at least one body bounding box. In other words, when at least part of the area that delimits the detectable portion of the body of the person overlaps the pillow area or vice versa.

In some embodiments, the computing system determines that there are no persons within the at least one image when the face detection algorithm and the body detection algorithm at step iii) do not generate any face bounding box and body bounding box in the at least one image. That is to say, since the face detection algorithm and the body detection algorithm are not able to detect a face or body, respectively, within the image, it means that there is nobody within the area covered by the imaging device that captures or generates the images, nor in bed or in the area surrounding the bed.

In some embodiments, the computing system determines the pose of the bedridden or bed-resting person determined at step vii) of the method based on a position of the key landmarks points detected at step iv) of the method relative to the corresponding face bounding box. Preferably, the computing system may determine the pose of the bedridden or bed-resting person based on only the key landmark point that corresponds to the nose of the person.

In some embodiments, the computing system determines that there is a person within the at least one image but the person is not bedridden or bed-resting when it is determined that the face boundary box and the body bounding box are out of the first and second areas. So, when the face boundary boxes and the body bounding boxes generated do not overlap with the first and second areas, areas that delimit the bed, is because the detected person is not in bed. This means that the detected person may be the bedridden or bed-resting person but he is standing up or he has fallen out of bed or he may be a visit, such as a relative, a health staff, a doctor, etc.

In some embodiments, when the computing system determines that there is a first person who is bedridden or bed-resting in the at least one image and it also determines that there is at least one second person in the at least one image but this second person(s) is not bedridden or bed-resting, it can be established that the at least one second person is a visit.

In some embodiments, when it has been determined that there is a person within the at least one image but the person is not bedridden or bed-resting, the computing system applies a body keypoints detection algorithm on the at least one image to detect body key points inside the generated at least one body bounding box. Depending on the body keypoints detection algorithm used a different set of body key points can be generated. For example, these body keypoints may correspond with the neck, shoulders, elbows, wrists, hip, knees and ankles, among others, of the detected person. The body keypoints detection algorithms used may be any algorithm well-known in the state of the art, such as those cited in the background of this document

In some embodiments, the computing system estimates the body pose of the person based on the detected body key points. This estimation can be carried out by a body pose model that may determine, based on the location of the detected body keypoints related to each other and to the corresponding body boundary box. There exist multiple body pose models and classifiers well-known in the state of the art as those cited in the background of this document.

In some embodiments, the computing system determines that there is a person attempting the get out the bed when there are no key landmark points within the first or the second area and when there is at least one point belonging to the at least one body bounding box within the second area and the first area is free from the at least one face bounding box and the at least one body bounding box. In other words, the computing system may determine that there is a person attempting the get out the bed when only detects the presence of the body of the person within the second area.

In some embodiments, the face detection algorithm and the body detection algorithm are applied substantially simultaneously on the at least one image.

In some embodiments, the method for monitoring bedridden and bed-resting persons is carried out for a plurality of received top view images of a same bed, so that it is determined the body pose of the person as the most frequent body pose obtained from each one of the plurality of images.

In some embodiments, the computing system may, in response to detecting that the bedridden or bed-resting person has fallen to the floor or is attempting to get out the bed, generate an event or alarm to inform a user, generally health staff, about the situation so the bedridden or bed-resting person can be duly attended.

A second object of the present invention is a computer program which, when executed by a computer, causes the computer to perform the computer-implemented method previously described. The computer may be a desktop computer, laptop (or notebook) computer, workstation, tablet computer, mobile phone, smart device, switch, router, server, blade enclosure, or any other processing device or equipment including a processing resource. In examples described herein, a processing resource may include, for example, one processor or multiple processors included in a single computing device or distributed across multiple computing devices.

A third object of the present invention is a non-transitory computer-readable storage medium storing a computer program as herein described. As used herein, a "non-transitory computer-readable storage medium" may be any electronic, magnetic, optical, or other physical storage apparatus to contain or store information such as executable instructions, data, and the like. For example, any non-transitory computer-readable storage medium described herein may be any of Random Access Memory (RAM), volatile memory, non-volatile memory, flash memory, a storage drive (e.g., a hard drive), a solid-state drive, any type of storage disc (e.g., a compact disc, a DVD, etc.), and the like, or a combination thereof.

A fourth object of the invention is a system for monitoring bedridden and bed-resting people. The system comprises a monitoring system adapted for being installed above at least one bed, preferably on the ceiling above the bed, which is arranged for being used by a bedridden or bed-resting person, the monitoring system comprising at least one imaging device which is configured to capture or generate top view images of the at least one bed. The imaging devices may be cameras, video cameras, time-of-flight (TOF) cameras, depth sensors, or any other imaging device capable of directly gathering images or generating them. The monitoring system may comprise a processing unit to generate said images from those imaging devices not able to directly capture said images (e.g., TOF-cameras or depth sensors).

The system further comprises a computing system comprising:
a receiving module configured to receive the at least one top view image of the at least one bed captured or generated by the corresponding imaging device;
a face detection module configured to execute face detection algorithms;
a body detection module configured to compute body detection algorithms;
a facial landmarks detection module configured to compute face landmarks detection algorithms; and
processing means configured to:
   delimit a first area corresponding to the pillow area and a second area corresponding to the rest of the bed on the at least one top view image;
   generate, by using the face detection module and the body detection module, respective face bounding boxes and body bounding boxes, respectively, on the at least one image, the face bounding boxes containing a detectable portion of the face of persons and the body bounding boxes containing a detectable portion of bodies of the persons in the at least one image;
   detect, by using the facial landmarks detection module and when at least one face bounding box has been generated, key landmark points inside the corresponding at least one face bounding box;
   check, when at least one face bounding box has been generated, a position of the detected key landmark points and of corners of the at least one face bounding box relative to the first area and the second area;
   check, when at least one body bounding box has been generated, the position of the at least one body bounding box relative to the first area;
   determine that there is a bedridden or bed-resting person in the at least one image when:
      at least one of the key landmark points is located within the first or second area;
         and/or
      at least one point belonging to the generated at least one body bounding box is located within the first area or when at least one point belonging to the first area is located within the generated at least one body bounding box.

In some embodiments, the system comprises a body keypoints detection module configured to execute body keypoints detection algorithms so as to detect keypoints inside the generated at least one body bounding box to estimate the body pose of the person.

In some embodiments, the system comprises a body pose classifying module configured to execute pose classification models so as to determine whether the person is laying on the floor based on the estimated body pose of the person.

The method and system herein described provide a solution that is able to combine different computer vision detection methods to detect in a robust, reliable and accurate manner the following events: whether the person is in the bed and his in-bed body pose, whether the person is attempting to get out the bed, whether the person has left the room where the bed is located, whether the person has received a visit, or whether the person has fallen to the floor.

### BRIEF DESCRIPTION OF THE DRAWINGS

To complete the description and in order to provide a better understanding of the invention, a set of drawings is provided. Said drawings form an integral part of the description and illustrate embodiments of the invention, which should not be interpreted as restricting the scope of the invention, but just as an example of how the invention can be carried out. The drawings comprise the following figures:
Fig. 1 shows a flow diagram of a method for monitoring bedridden and bed-resting people, according to an embodiment of the invention.
Figure 2 shows a flow diagram that includes some additional method steps for the computer-implemented method for monitoring bedridden and bed-resting people of Figure 1, according to an embodiment of the invention.
Figures 3A to 3E show five top view images received at the computing system in which different situations which are detected by the method for monitoring bedridden and bed-resting persons of Figures 1 and 2 are depicted.
Fig. 4 shows a block diagram of a system for monitoring bedridden and bed-resting people, according to an embodiment of the invention.

### DESCRIPTION OF A MODE OF EMBODIMENT OF THE INVENTION

In the following description, for purposes of explanation, numerous specific details are set forth in order to provide a thorough understanding of the present systems and methods. It will be apparent, however, to one skilled in the art that the present systems and methods may be practiced without these specific details. Reference in the specification to "an example", "an embodiment" or similar language means that a particular feature, structure, or characteristic described in connection with that example or embodiments is included as described but may not be included in other examples or embodiments.

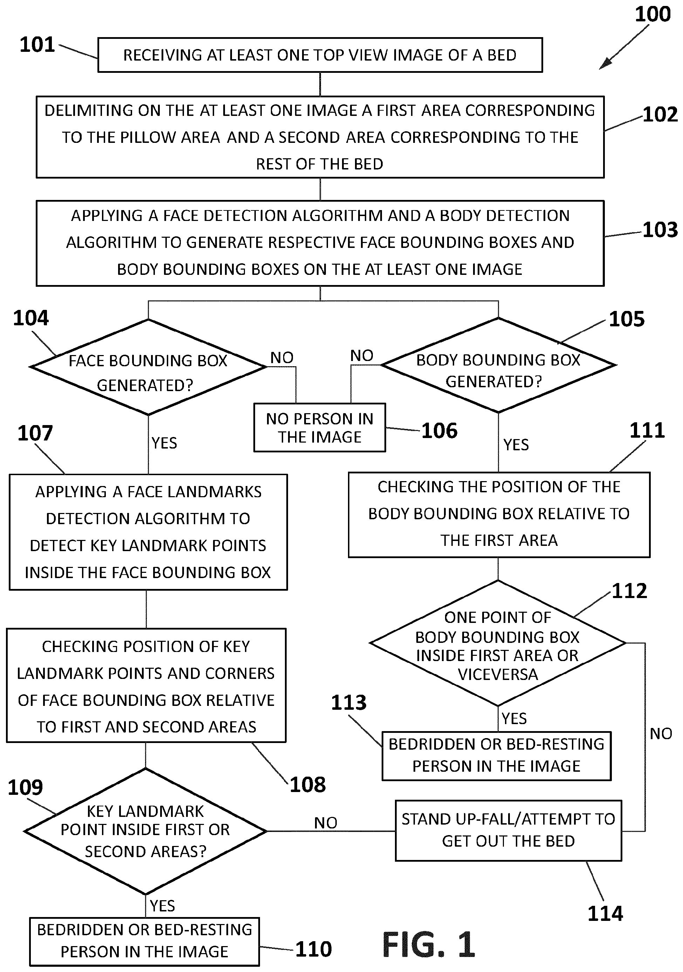

Fig. 1 shows a flow diagram of a computer-implemented method 100 for monitoring bedridden and bed-resting people, according to an embodiment of the invention.

At step 101 of the method 100, at least one top view image of a particular bed is received at the computing system.

At step 102 of the method 100, the received image is processed by the computing system to delimit a first area corresponding to the pillow area and a second area corresponding to the rest of the bed in the image. The computing system may use an object recognition algorithm to detect these two areas within the bed and draw a polygonal line that confines them. The combination of these two areas delimits the outer borders of the bed. In some embodiments, the first and second areas can be manually drawn on the images and provided to the computing system.

At step 103 of the method 100, the computing system applies a face detection algorithm and a body detection algorithm to generate respective face bounding boxes and body bounding boxes on the at least one image. The face detection algorithm detects the presence of faces within the image and draws a polygonal line around the detectable portion of the faces detected such that said detectable portions are contained within said face bounding boxes. Similarly, the body detection algorithm detects the presence of bodies in the image and draws a polygonal line around the detectable portions of said bodies.

At step 104 of the method 100, it is checked whether a face bounding box has been generated by the face detection algorithm and if so, at step 107 of the method, the computing system applies a face landmarks detection algorithm on the image to detect key landmark points inside said face bounding box. These face landmarks detection algorithm mark on the image specific elements of the face of the person, such as the nose, eyes or mouth, among others. Then, at step 108 of the method 100, the computing system checks the position of the detected key landmark points and of the corners of the face bounding box relative to the first area and to the second area within the image. In this way, the computing system can stablish 109 whether some of said key landmark point are inside or outside the first and second areas, i.e., inside or outside the bed. When there is at least one key landmark point within the first or second area, the computing system determines, at step 110 of the method 100, that the person in the image, from whom the face boundary box has been obtained, is in bed (he is bedridden or bed-resting). If none of the key landmark points is within the first or second area, the computing system determines, at step 114 of the method 100, that the person is not in the bed. Thus, this person could be the bedridden/bed-resting person who has fallen to the floor or who is standing near the bed or may be the bedridden person attempting to get out the bed. The computing system can also determine the pose of the bedridden or bed-resting person based on the position of the key landmarks points relative to the corresponding face bounding box

At step 105 of the method 100, it is checked whether a body bounding box has been generated by the body detection algorithm and if so, at step 111 of the method 100, the computing system checks the position of the body bounding box relative to the first area. In this way, the computing system can stablish 112 whether the points within the body bounding box are inside or outside first area, i.e., the pillow area, and vice versa. When there is at least one point of the body bounding box within the first area or at least one point of the first area within the body bounding box, the computing system determines, at step 113 of the method 100, that the person in the image, from whom the body boundary box has been obtained, is in bed (he is bedridden or bed-resting). If none of the points of the body bounding box are within the first area and vice versa, the computing system determines, at step 114 of the method 100, that the person is not in the bed. Thus, this detected person may be the bedridden/bed-resting person who has fallen to the floor or who is standing near the bed or may be the bedridden person attempting to get out the bed.

When the computing system determines at steps 104 and 105 of the method 100 that neither face bounding boxes or body bounding boxes have been generated, it determines, at step 106 of the method 100, that there is nobody within the image. The computing system may trigger an alarm in those cases in which there should be a bedridden person or bed-resting person in the monitored bed.

The computing system may determine that there is a visit in the image, the visit being any other person in the image that is not the bedridden or bed-resting person, when at steps 110 or 113 of the method 100 it is determined that a first person in the image is bedridden or bed-resting and when a body bounding box associated to a second person in the image is located out of the first and second areas, i.e., out of the bed. Besides, the computing system may further determine that there is a visit in the image when at steps 110 or 113 of the method 100 it is determined that a first person in the image is bedridden or bed-resting and when all the key landmark points inside the face bounding box associated to said second person in the image are out of the first or second area, i.e, out of the bed.

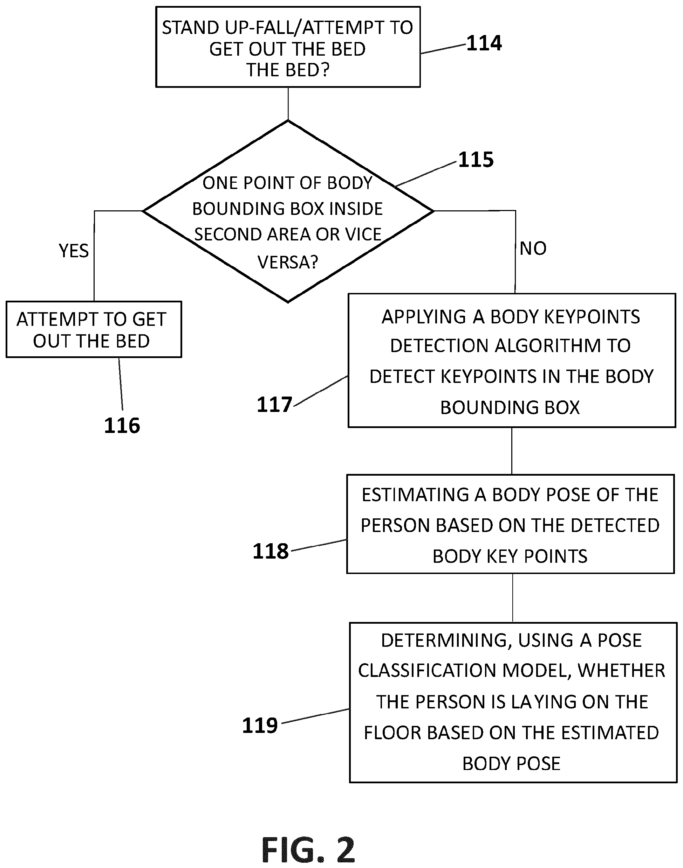

Figure 2 shows a flow diagram that includes some additional method steps for the computer-implemented method 100 for monitoring bedridden and bed-resting people of Figure 1.

When at step 114 of the method 100, the computing system determines that the person detected in the image is not in the bed so, this person could be a visit or may be the bedridden/bed-resting person who has fallen to the floor or who is standing near the bed or may be the bedridden person attempting to get out the bed, the computing system checks 115 whether the points within the body bounding box are inside or outside second area, i.e., the bed excluding the pillow area, and vice versa. If the computing system determines that there are no key landmark points within the first or the second area and that there is at least one point of the body bounding box inside the second area or vice versa, then it is determined 116 that the person is attempting to get out the bed. For example, the person may be sitting at the border of the bed and trying to get up. Then, the computing system may trigger an alarm to warn the health staff. If the computing system determines, at step 115 of the method 100, that there are no points of the body bounding box inside the second area and vice versa, then it is determined that the person is out the bed, and it is checked its body pose.

At step 117 of the method 100, the computing system applies a body keypoints detection algorithm to detect body key points inside the generated at least one body bounding box. For example, these body keypoints may correspond with the neck, shoulders, elbows, wrists, hip, knees and ankles of the detected person, among many other body keypoints. At step 118 of the method 100, the computing system estimates the body pose of the person based on the detected body keypoints. That is to say, an estimation of the body pose of the person can be made based on the position of the body keypoints relative to each other and relative to the body bounding box. After that, at step 119 of the method 100, the computing system using a pose classification model determines whether the person is laying on the floor based on the estimated body pose previously obtained. The person may have fallen to the floor or may be standing up next to the bed. If the person has fallen to the floor, the computing system may trigger an alarm. If the person is standing up and he is the only person within the image, the system may trigger an alarm in those cases in which there should be a bedridden person or bed-resting person in the monitored bed.

While figures 1 and 2 shows two flowcharts with the steps of the method in a particular order, in some other embodiments, the order in which these steps are carried out may be different.

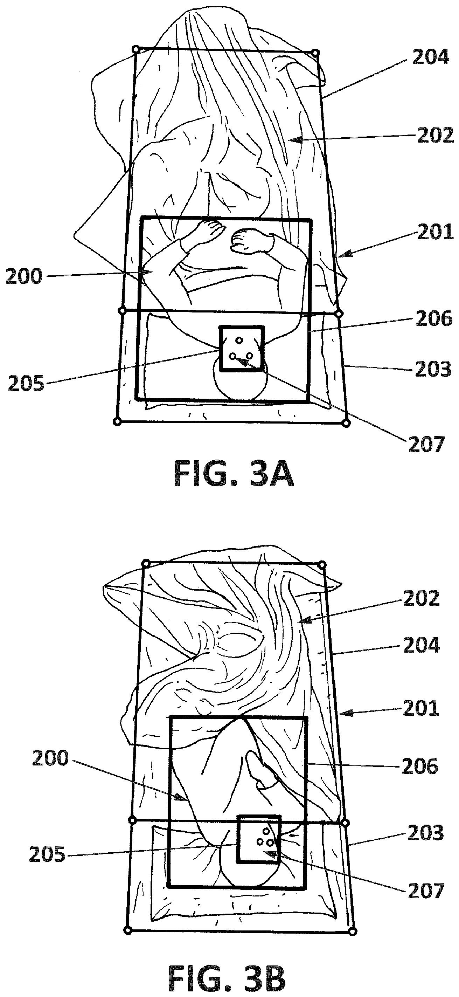

Figures 3A to 3E show five top view images received at the computing system in which different situations which are detected by the method for monitoring bedridden and bed-resting persons of Figures 1 and 2 are depicted.

Figure 3A shows a top view image of a bedridden person 200 laying on his back in a bed 201 and being partially covered with sheets 202. In this image, the first area 203, the second area 204, the face bounding box 205, the body bounding box 206 and the face landmarks points 207 are drawn in the image. In this image, the body detection algorithm is only able to detect the visible portion of the body of the person 200.

Figure 3B shows a top view image of a bedridden person 200 laying on his side in the bed 201 and being partially covered with sheets 202. In this case, the computing system is able to determine that the person is lying on his right side based on a position of the detected key landmarks points 207 relative to the face bounding box 205. In some embodiments, the computing system may be able to determine that is the body pose o the person, left-to-right, top-down, based on only the key landmark point that corresponds to the nose of the person and its position relative to the face bounding box 205.

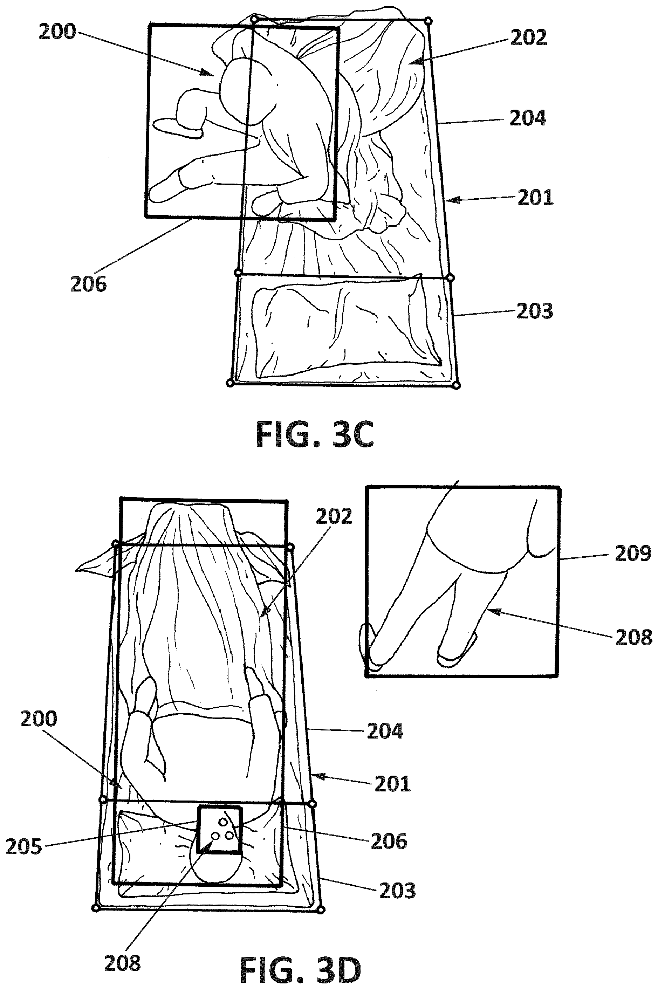

Figure 3C shows a top view image of a bedridden person 200 attempting to get of the bed 201. In this particular case, the computing system does not generate a face bounding box since there is no detectable portion of the face within the image. Therefore, there are no key landmark points within the first area 203 or the second area 204. In such case, the body bounding box 206 is detected partially overlapping the second area 204 and thus, the computing system determines that the person 200 is attempting to get out the bed 201.

Figure 3D shows a top view image of a bedridden person 200 laying on his back in a bed 201 and being partially covered with sheets 202 with a visit 208. In this case, the computing system detects the person 200 lying on his bed as in figure 3A, but it also detects a second body bounding box 209 that does not overlap with the first area 205 or the second area 206. Since the computing system has already detected a person 200 in the bed 201, this second person 208 has to be a visit, such as a relative, a health staff, a doctor, etc. In this image, the body detection algorithm is able to detect the presence of the legs of the person 200 under the sheets 202 and identify their contour so the body bounding box 209 includes the visible and the covered portion of the body.

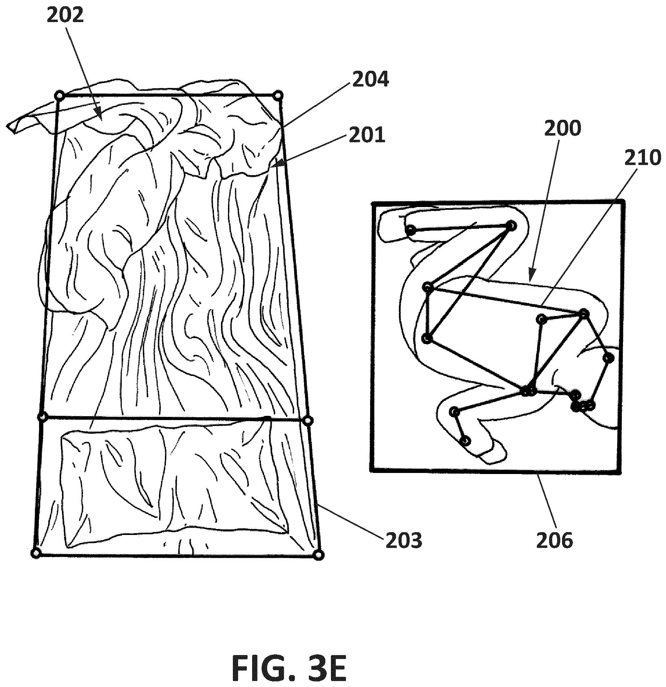

Figure 3E shows a top view image of a bedridden person 200 who has fallen to the floor. The computing system detects that the body bounding box 206 is out of the first area 203 and of the second area 204 and generates the body keypoints 210 inside the body bounding box 206. In this particular case, the keypoints 210 are located in the head, shoulders, back, elbows, wrists, hip, knees and ankles of the person 200. With these keypoints 210 the body pose of the person 200 is estimated and then, using a pose classification model and according to the estimated body pose, it is determined that the person 200 is laying on the floor.

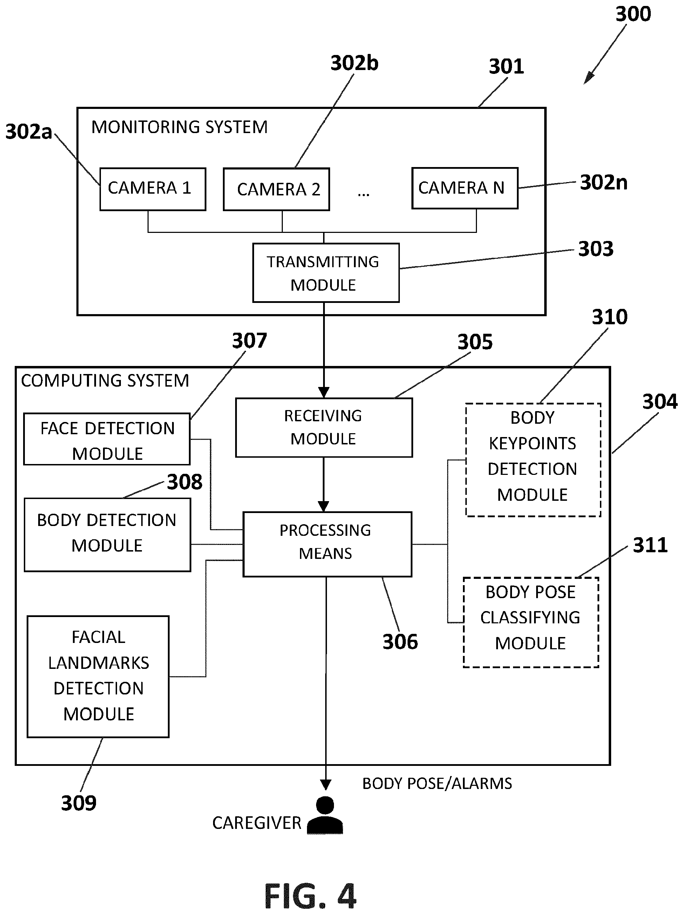

Fig. 4 shows a block diagram 300 of a system for monitoring bedridden and bed-resting people, according to an embodiment of the invention. It should be understood that the system 300 may include additional components and that some of the components described herein may be removed and/or modified. Additionally, implementation of the example system 300 is not limited to such example as shown in Figure 4.

The system 300 comprises a monitoring system 301 adapted for being installed inside a room with one or more beds and it is formed by a number of cameras 302a-n. Preferably, these cameras will be installed into the ceiling of the room and oriented towards the beds. Preferably there will be one single camera per bed. For those cases in which the monitoring system 301 is formed by imaging devices (e.g., TOF-cameras or depth sensors) that are not able to directly capture said images but to capture data form which said images are to be generated, the monitoring system 301 may further comprises a processing unit (not shown in this figure) to locally generate the images. Alternatively, said captured raw data may be sent to the computing system 304 where the images may be generated.

The images or raw data captured by the monitoring system 301 is sent by a transmitting module 303 to a receiving module 305 of the computing system 304. The communications between the transmitting module 303 and the receiving module 305 may be wired or may be a wireless communication using GPRS, WiFi, Bluetooth, or any other wireless protocol.

The computing system 304 further comprises processing means 306 configured to delimit a first area corresponding to the pillow area and a second area corresponding to the rest of the bed on the at least one top view image and to generate, using the face detection module 307 that is configured to execute face detection algorithms and the body detection module 308 that is configured to execute body detection algorithms, respective face bounding boxes and body bounding boxes on the at least one image. The face bounding boxes contain the detectable portion of the faces of persons and the body bounding boxes contain the detectable portion of bodies of the persons in the at least one image.

The processing means 306 are further configured to detect, using the facial landmarks detection module 309 that is configured to execute facial landmarks detection algorithms and when at least one face bounding box has been generated, key landmark points inside the corresponding at least one face bounding box, check, when at least one face bounding box has been generated, a position of the detected key landmark points and of corners of the at least one face bounding box relative to the first area and the second area and check, when at least one body bounding box has been generated, the position of the at least one body bounding box relative to the first area.

Then, the processing means 306 determines that there is a bedridden or bed-resting person in the at least one image when:
at least one of the key landmark points is located within the first or second area; and/or
at least one point belonging to the generated at least one body bounding box is located within the first area or when at least one point belonging to the first area is located within the generated at least one body bounding box.

The computing system 304 also comprises a body keypoints detection module 310 configured to execute body keypoints detection algorithms so as to detect key points inside the generated at least one body box to estimate body pose of the person and a body pose classifying module 311 configured to execute pose classification models so as to determine whether the person is laying on the floor based on the estimated body pose of the person. The processing means 306 may send at any time the information about the body pose of the person being monitored to a central computing unit or a mobile devices, such as cellphones, PDAs, laptops, etc., of the caregivers in charge of these bedridden or bed-resting persons. When a fall, an attempt to get up of the bedridden or bed-resting person, an absence of the bedridden or bed-resting person or the presence of a visitor is detected, the processing means 306 may trigger and send an alarm to the caregivers so they can take care of the detected situation.

In this text, the term "comprises" and its derivations (such as "comprising", etc.) should not be understood in an excluding sense, that is, these terms should not be interpreted as excluding the possibility that what is described and defined may include further elements.

The invention is obviously not limited to the specific embodiments described herein, but also encompasses any variations that may be considered by any person skilled in the art within the general scope of the invention as defined in the claims.

## Claims

1. A computer-implemented method for monitoring bedridden and bed-resting persons, the method comprising the steps of:
i. receiving, at a computing system, at least one top view image of a bed;
ii. delimiting, by the computing system, on the at least one image a first area corresponding to an area of the bed where a pillow is located and a second area corresponding to the rest of the bed;
iii. applying, by the computing system, a face detection algorithm and a body detection algorithm to generate respective face bounding boxes and body bounding boxes in the at least one image, the face bounding boxes containing a detectable portion of the face of persons and the body bounding boxes containing a detectable portion of bodies of the persons in the at least one image, wherein a detectable portion of the face and a detectable portion of the body comprise, respectively, portions of the face and portions of the body that the face detection algorithm and the body detection algorithm are able to detect because they are visible or because the algorithms are able to estimate their presence, location and contour;
iv. applying, by the computing system and when the face detection algorithm has generated at least one face bounding box, a face landmarks detection algorithm to detect key landmark points inside the corresponding at least one face bounding box;
v. checking, by the computing system and when at least one face bounding box has been generated, a position of the detected key landmark points and of corners of the at least one face bounding box relative to the first area and the second area;
vi. checking, by the computing system and when at least one body box has been generated, the position of the at least one body bounding box relative to the first area;
vii. determining that there is a bedridden or bed-resting person in the at least one image when:
at least one of the key landmark points is located within the first or second area;
and/or
at least one point belonging to the generated at least one body bounding box is located within the first area or when at least one point belonging to the first area is located within the generated at least one body bounding box.

2. The computer-implemented method according to claim 1, comprising determining, by the computing system, that there are no persons within the at least one image when the face detection algorithm and the body detection algorithm at step iii) do not generate any face bounding box and body bounding box in the at least one image.

3. The computer-implemented method according to claim 1 or 2, comprising determining, by the computing system, a pose of the bedridden or bed-resting person determined at step vii) based on a position of the key landmarks points detected at step iv) relative to the corresponding face bounding box.

4. The computer-implemented method according to any one of the preceding claims, comprising determining, by the computing system, that there is a person within the at least one image out of the bed when it is determined that the face boundary box and the body bounding box are outside the first and second areas.

5. The computer-implemented method according to claim 4, comprising applying, by the computing system, a body keypoints detection algorithm to detect body key points inside the generated at least one body bounding box

6. The computer-implemented method according to claim 5, comprising estimating a body pose of the person based on the detected body key points.

7. The computer-implemented method according to claim 6, comprising determining, by the computing system and using a pose classification model, whether the person is laying on the floor based on the estimated body pose of the person.

8. The computer-implemented method according to any one of the preceding claims, comprising determining, by the computing system and there are no key landmark points within the first or the second area, that there is a person attempting the get out the bed when there is at least one point belonging to the at least one body bounding box within the second area and the first area is free from the at least one face bounding box and the at least one body bounding box.

9. The computer-implemented method according to any one of the preceding claims, wherein the face detection algorithm and the body detection algorithm are applied simultaneously on the at least one image.

10. The computer-implemented method according to any one of the preceding claims, comprising executing the method for monitoring the body pose of bedridden and bed-resting persons for a plurality of received top view images of a bed, and determining the body pose of the user as the most frequent body pose obtained for each one of the plurality of images.

11. A computer program which, when executed by a computer, causes the computer to perform the computer-implemented method according to any one of claims 1 to 10.

12. A non-transitory computer-readable storage medium storing a computer program in accordance with claim 11.

13. A system for monitoring bedridden and bed-resting people, comprising:
a monitoring system adapted for being installed above at least one bed which is arranged for being used by a bedridden or bed-resting person, the monitoring system comprising at least one imaging device, preferably a camera, which is configured to capture or generate top view images of the at least one bed;
**characterized in that** the system further comprises a computing system comprising:
a receiving module configured to receive the at least one top view image of the at least one bed captured or generated by the corresponding imaging device;
a face detection module configured to execute face detection algorithms;
a body detection module configured to compute body detection algorithms;
a facial landmarks detection module configured to compute face landmarks detection algorithms; and
processing means configured to:
delimit a first area corresponding to an area of the bed where a pillow is located and a second area corresponding to the rest of the bed on the at least one top view image;
generate, by using the face detection module and the body detection module, respective face bounding boxes and body bounding boxes, respectively, in the at least one image, the face bounding boxes containing a detectable portion of the face of persons and the body bounding boxes containing a detectable portion of bodies of the persons in the at least one image, wherein a detectable portion of the face and a detectable portion of the body comprise, respectively, portions of the face and portions of the body that the face detection algorithm and the body detection algorithm are able to detect because they are visible or because the algorithms are able to estimate their presence, location and contour;
detect, by using the facial landmarks detection module and when at least one face bounding box has been generated, key landmark points inside the corresponding at least one face bounding box;
check, when at least one face bounding box has been generated, a position of the detected key landmark points and of corners of the at least one face bounding box relative to the first area and the second area;
check, when at least one body box has been generated, the position of the at least one body bounding box relative to the first area;
determine that there is a bedridden or bed-resting person in the at least one image when:
at least one of the key landmark points is located within the first or second area;
and/or
at least one point belonging to the generated at least one bounding box is located within the first area or when at least one point belonging to the first area is located within the generated at least one body bounding box.

14. The system according to claim 13, comprising a body keypoints detection module configured to execute body keypoints detection algorithms so as to detect key points inside the generated at least one body box to estimate body pose of the person.

15. The system according to claim 14, comprising a body pose classifying module configured to execute pose classification models so as to determine whether the person is laying on the floor based on the estimated body pose of the person.

## Patentansprüche

1. Computerimplementiertes Verfahren zur Überwachung von bettlägerigen und bettruhenden Personen, wobei das Verfahren die folgenden Schritte umfasst:
i. Empfangen, an einem Rechensystem, mindestens eines Draufsichtbildes eines Bettes;
ii. Festlegen, durch das Rechensystem, auf dem mindestens einen Bild eines ersten Bereichs, der einem Bereich des Bettes entspricht, in dem sich ein Kopfkissen befindet, und eines zweiten Bereichs, der dem Rest des Bettes entspricht;
iii. Anwenden, durch das Rechensystem, eines Gesichtserkennungsalgorithmus und eines Körpererkennungsalgorithmus, um entsprechende Gesichts-Begrenzungsrahmen und Körper-Begrenzungsrahmen in dem mindestens einen Bild zu erzeugen, wobei die Gesichts-Begrenzungsrahmen einen erkennbaren Abschnitt des Gesichts von Personen enthalten und die Körper-Begrenzungsrahmen einen erkennbaren Abschnitt von Körpern der Personen in dem mindestens einen Bild enthalten, wobei ein erkennbarer Abschnitt des Gesichts und ein erkennbarer Abschnitt des Körpers jeweils Abschnitte des Gesichts und Abschnitte des Körpers umfassen, die der Gesichtserkennungsalgorithmus und der Körpererkennungsalgorithmus erkennen können, weil sie sichtbar sind oder weil die Algorithmen in der Lage sind, ihre Anwesenheit, Position und Kontur abzuschätzen;
iv. Anwenden, durch das Rechensystem und wenn der Gesichtserkennungsalgorithmus mindestens einen Gesichts-Begrenzungsrahmen erzeugt hat, eines Gesichts-Landmarken-Erkennungsalgorithmus, um Schlüssel-Landmarken-Punkte innerhalb des entsprechenden mindestens einen Gesichts-Begrenzungsrahmens zu erkennen;
v. Überprüfen, durch das Rechensystem und wenn mindestens ein Gesichts-Begrenzungsrahmen erzeugt wurde, einer Position der erkannten Schlüssel-Landmarken-Punkte und von Ecken des mindestens einen Gesichts-Begrenzungsrahmens relativ zum ersten Bereich und zum zweiten Bereich;
vi. Überprüfen, durch das Rechensystem und wenn mindestens ein Körper-Begrenzungsrahmen erzeugt wurde, der Position des mindestens einen Körper-Begrenzungsrahmens relativ zum ersten Bereich;
vii. Bestimmen, dass sich eine bettlägerige oder bettruhende Person in dem mindestens einen Bild befindet, wenn:
mindestens einer der Schlüssel-Landmarken-Punkte innerhalb des ersten oder zweiten Bereichs liegt;
und/oder
mindestens ein Punkt, der zu dem erzeugten mindestens einen Körper-Begrenzungsrahmen gehört, innerhalb des ersten Bereichs liegt, oder wenn mindestens ein Punkt, der zum ersten Bereich gehört, innerhalb des erzeugten mindestens einen Körper-Begrenzungsrahmens liegt.

2. Computerimplementiertes Verfahren nach Anspruch 1, umfassend das Bestimmen, durch das Rechensystem, dass sich keine Personen in dem mindestens einen Bild befinden, wenn der Gesichtserkennungsalgorithmus und der Körpererkennungsalgorithmus in Schritt iii) keinen Gesichts-Begrenzungsrahmen und keinen Körper-Begrenzungsrahmen in dem mindestens einen Bild erzeugen.

3. Computerimplementiertes Verfahren nach Anspruch 1 oder 2, umfassend das Bestimmen, durch das Rechensystem, einer Pose der in Schritt vii) bestimmten bettlägerigen oder bettruhenden Person basierend auf einer Position der in Schritt iv) erkannten Schlüssel-Landmarken-Punkte relativ zu dem entsprechenden Gesichts-Begrenzungsrahmen.

4. Computerimplementiertes Verfahren nach einem der vorhergehenden Ansprüche, umfassend das Bestimmen, durch das Rechensystem, dass sich eine Person in dem mindestens einen Bild außerhalb des Bettes befindet, wenn bestimmt wird, dass der Gesichts-Begrenzungsrahmen und der Körper-Begrenzungsrahmen außerhalb des ersten und zweiten Bereichs liegen.

5. Computerimplementiertes Verfahren nach Anspruch 4, umfassend das Anwenden, durch das Rechensystem, eines Körper-Schlüsselpunkt-Erkennungsalgorithmus, um Körper-Schlüsselpunkte innerhalb des erzeugten mindestens einen Körper-Begrenzungsrahmens zu erkennen.

6. Computerimplementiertes Verfahren nach Anspruch 5, umfassend das Schätzen einer Körperpose der Person basierend auf den erkannten Körper-Schlüsselpunkten.

7. Computerimplementiertes Verfahren nach Anspruch 6, umfassend das Bestimmen, durch das Rechensystem und unter Verwendung eines Posen-Klassifizierungsmodells, ob die Person auf dem Boden liegt, basierend auf der geschätzten Körperpose der Person.

8. Computerimplementiertes Verfahren nach einem der vorhergehenden Ansprüche, umfassend das Bestimmen, durch das Rechensystem und wenn sich keine Schlüssel-Landmarken-Punkte innerhalb des ersten oder des zweiten Bereichs befinden, dass eine Person versucht, aus dem Bett aufzustehen, wenn mindestens ein Punkt, der zu dem mindestens einen Körper-Begrenzungsrahmen gehört, innerhalb des zweiten Bereichs liegt und der erste Bereich frei von dem mindestens einen Gesichts-Begrenzungsrahmen und dem mindestens einen Körper-Begrenzungsrahmen ist.

9. Computerimplementiertes Verfahren nach einem der vorhergehenden Ansprüche, wobei der Gesichtserkennungsalgorithmus und der Körpererkennungsalgorithmus gleichzeitig auf das mindestens eine Bild angewendet werden.

10. Computerimplementiertes Verfahren nach einem der vorhergehenden Ansprüche, umfassend das Ausführen des Verfahrens zur Überwachung der Körperpose von bettlägerigen und bettruhenden Personen für eine Vielzahl von empfangenen Draufsichtbildern eines Bettes und Bestimmen der Körperpose des Benutzers als die häufigste Körperpose, die für jedes der Vielzahl von Bildern erhalten wurde.

11. Computerprogramm, das, wenn es von einem Computer ausgeführt wird, den Computer veranlasst, das computerimplementierte Verfahren nach einem der Ansprüche 1 bis 10 durchzuführen.

12. Nichtflüchtiges computerlesbares Speichermedium, das ein Computerprogramm nach Anspruch 11 speichert.

13. System zur Überwachung von bettlägerigen und bettruhenden Personen, umfassend:
ein Überwachungssystem, das dazu angepasst ist, über mindestens einem Bett installiert zu werden, das zur Nutzung durch eine bettlägerige oder bettruhende Person vorgesehen ist, wobei das Überwachungssystem mindestens eine Bildgebungsvorrichtung, vorzugsweise eine Kamera, umfasst, die konfiguriert ist, Draufsichtbilder des mindestens einen Bettes zu erfassen oder zu erzeugen;
**dadurch gekennzeichnet, dass** das System ferner ein Rechensystem umfasst, umfassend:
ein Empfangsmodul, das konfiguriert ist, das mindestens eine Draufsichtbild des mindestens einen Bettes zu empfangen, das von der entsprechenden Bildgebungsvorrichtung erfasst oder erzeugt wurde;
ein Gesichtserkennungsmodul, das konfiguriert ist, Gesichtserkennungsalgorithmen auszuführen;
ein Körpererkennungsmodul, das konfiguriert ist, Körpererkennungsalgorithmen zu berechnen;
ein Modul zur Erkennung von Gesichts-Landmarken, das konfiguriert ist, Algorithmen zur Erkennung von Gesichts-Landmarken zu berechnen; und
Verarbeitungsmittel, die konfiguriert sind zum:
Festlegen eines ersten Bereichs, der einem Bereich des Bettes entspricht, in dem sich ein Kopfkissen befindet, und eines zweiten Bereichs, der dem Rest des Bettes auf dem mindestens einen Draufsichtbild entspricht;
Erzeugen, unter Verwendung des Gesichtserkennungsmoduls und des Körpererkennungsmoduls, entsprechender Gesichts-Begrenzungsrahmen bzw. Körper-Begrenzungsrahmen in dem mindestens einen Bild, wobei die Gesichts-Begrenzungsrahmen einen erkennbaren Abschnitt des Gesichts von Personen enthalten und die Körper-Begrenzungsrahmen einen erkennbaren Abschnitt von Körpern der Personen in dem mindestens einen Bild enthalten, wobei ein erkennbarer Abschnitt des Gesichts und ein erkennbarer Abschnitt des Körpers jeweils Abschnitte des Gesichts und Abschnitte des Körpers umfassen, die der Gesichtserkennungsalgorithmus und der Körpererkennungsalgorithmus erkennen können, weil sie sichtbar sind oder weil die Algorithmen in der Lage sind, ihre Anwesenheit, Position und Kontur abzuschätzen;
Erkennen, unter Verwendung des Moduls zur Erkennung von Gesichts-Landmarken und wenn mindestens ein Gesichts-Begrenzungsrahmen erzeugt wurde, von Schlüssel-Landmarken-Punkten innerhalb des entsprechenden mindestens einen Gesichts-Begrenzungsrahmens;
Überprüfen, wenn mindestens ein Gesichts-Begrenzungsrahmen erzeugt wurde, einer Position der erkannten Schlüssel-Landmarken-Punkte und von Ecken des mindestens einen Gesichts-Begrenzungsrahmens relativ zum ersten Bereich und zum zweiten Bereich;
Überprüfen, wenn mindestens ein Körper-Begrenzungsrahmen erzeugt wurde, der Position des mindestens einen Körper-Begrenzungsrahmens relativ zum ersten Bereich;
Bestimmen, dass sich eine bettlägerige oder bettruhende Person in dem mindestens einen Bild befindet, wenn:
mindestens einer der Schlüssel-Landmarken-Punkte innerhalb des ersten oder zweiten Bereichs liegt;
und/oder
mindestens ein Punkt, der zu dem erzeugten mindestens einen Begrenzungsrahmen gehört, innerhalb des ersten Bereichs liegt, oder wenn mindestens ein Punkt, der zum ersten Bereich gehört, innerhalb des erzeugten mindestens einen Körper-Begrenzungsrahmens liegt.

14. System nach Anspruch 13, umfassend ein Körper-Schlüsselpunkt-Erkennungsmodul, das konfiguriert ist, Körper-Schlüsselpunkt-Erkennungsalgorithmen auszuführen, um Schlüsselpunkte innerhalb des erzeugten mindestens einen Körper-Begrenzungsrahmens zu erkennen, um die Körperpose der Person zu schätzen.

15. System nach Anspruch 14, umfassend ein Posen-Klassifizierungsmodul, das konfiguriert ist, Posen-Klassifizierungsmodelle auszuführen, um basierend auf der geschätzten Körperpose der Person zu bestimmen, ob die Person auf dem Boden liegt.

## Revendications

1. Méthode mise en œuvre par ordinateur pour surveiller des personnes alitées et en repos au lit, la méthode comprenant les étapes suivantes :
i. au niveau d'un système informatique, recevoir au moins une image en vue de dessus d'un lit ;
ii. sur l'au moins une image, délimiter par le système informatique une première zone correspondant à une zone du lit où se trouve un oreiller et une deuxième zone correspondant au reste du lit ;
iii. appliquer par le système informatique un algorithme de détection de visage et un algorithme de détection de corps pour générer des boîtes englobantes de visage et des boîtes englobantes de corps respectives dans l'au moins une image, les boîtes englobantes de visage contenant une partie détectable du visage des personnes et les boîtes englobantes de corps contenant une partie détectable des corps des personnes dans l'au moins une image, dans laquelle une partie détectable du visage et une partie détectable du corps comprennent respectivement des parties du visage et des parties du corps que l'algorithme de détection de visage et l'algorithme de détection de corps sont en mesure de détecter parce qu'elles sont visibles ou parce que les algorithmes sont en mesure d'estimer leur présence, leur emplacement et leur contour ;
iv. lorsque l'algorithme de détection de visage a généré au moins une boîte englobante de visage, appliquer par le système informatique un algorithme de détection de repères faciaux afin de détecter des points de repère clés à l'intérieur de l'au moins une boîte englobante de visage correspondante ;
v. lorsqu'au moins une boîte englobante de visage a été générée, vérifier par le système informatique une position des points de repère clés détectés et des coins de l'au moins une boîte englobante de visage par rapport à la première zone et à la deuxième zone ;
vi. lorsqu'au moins une boîte de corps a été générée, vérifier par le système informatique la position de l'au moins une boîte englobante de corps par rapport à la première zone ;
vii. déterminer la présence d'une personne alitée ou en repos au lit dans l'au moins une image :
lorsqu'au moins un des points de repère clés est situé à l'intérieur de la première zone ou de la deuxième zone ;
et/ou
lorsqu'au moins un point appartenant à l'au moins une boîte englobante de corps générée est situé à l'intérieur de la première zone ou lorsqu'au moins un point appartenant à la première zone est situé à l'intérieur de l'au moins une boîte englobante de corps générée.

2. Méthode mise en œuvre par ordinateur selon la revendication 1, comprenant le fait de déterminer par le système informatique qu'aucune personne ne se trouve dans l'au moins une image lorsque l'algorithme de détection de visage et l'algorithme de détection de corps à l'étape iii) ne génèrent aucune boîte englobante de visage et aucune boîte englobante de corps dans l'au moins une image.

3. Méthode mise en œuvre par ordinateur selon la revendication 1 ou 2, comprenant le fait de déterminer par le système informatique une posture de la personne alitée ou en repos au lit déterminée à l'étape vii) sur la base d'une position des points de repère clés détectés à l'étape iv) par rapport à la boîte englobante de visage correspondante.

4. Méthode mise en œuvre par ordinateur selon l'une quelconque des revendications précédentes, comprenant le fait de déterminer par le système informatique la présence d'une personne hors du lit dans l'au moins une image lorsqu'il est déterminé que la boîte englobante de visage et la boîte englobante de corps se trouvent en dehors des première et deuxième zones.

5. Méthode mise en œuvre par ordinateur selon la revendication 4, comprenant l'application par le système informatique d'un algorithme de détection de points clés de corps pour détecter des points clés de corps à l'intérieur de l'au moins une boîte englobante de corps générée.

6. Méthode mise en œuvre par ordinateur selon la revendication 5, comprenant l'estimation d'une posture corporelle de la personne sur la base des points clés de corps détectés.

7. Méthode mise en œuvre par ordinateur selon la revendication 6, comprenant le fait de déterminer par le système informatique si la personne est allongée sur le sol en utilisant un modèle de classification de postures, sur la base de la posture corporelle estimée de la personne.

8. Méthode mise en œuvre par ordinateur selon l'une quelconque des revendications précédentes, comprenant le fait de déterminer par le système informatique, en l'absence de points de repère clés dans la première ou la deuxième zone, qu'une personne tente de sortir du lit lorsqu'il existe au moins un point appartenant à l'au moins une boîte englobante de corps dans la deuxième zone et que la première zone ne contient pas l'au moins une boîte englobante de visage et l'au moins une boîte englobante de corps.

9. Méthode mise en œuvre par ordinateur selon l'une quelconque des revendications précédentes, dans laquelle l'algorithme de détection de visage et l'algorithme de détection de corps sont appliqués simultanément sur l'au moins une image.

10. Méthode mise en œuvre par ordinateur selon l'une quelconque des revendications précédentes, comprenant le fait d'exécuter la méthode pour surveiller la posture corporelle de la personne alitée et en repos au lit pour une pluralité d'images en vue de dessus reçues d'un lit, et de déterminer la posture corporelle de l'utilisateur comme étant la posture corporelle la plus fréquente obtenue pour chacune de la pluralité d'images.

11. Programme informatique qui, lorsqu'il est exécuté par un ordinateur, amène l'ordinateur à mettre en œuvre la méthode mise en œuvre par ordinateur selon l'une quelconque des revendications 1 à 10.

12. Support de stockage non transitoire lisible par ordinateur stockant un programme informatique conformément à la revendication 11.

13. Système de surveillance de personnes alitées et en repos au lit, comprenant :
un système de surveillance conçu pour être installé au-dessus d'au moins un lit agencé pour être utilisé par une personne alitée ou en repos au lit, le système de surveillance comprenant au moins un dispositif d'imagerie, de préférence une caméra, qui est configuré pour capturer ou générer des images en vue de dessus de l'au moins un lit ;
**caractérisé en ce que** le système comprend en outre un système informatique comprenant :
un module de réception configuré pour recevoir l'au moins une image en vue de dessus de l'au moins un lit, capturée ou générée par le dispositif d'imagerie correspondant ;
un module de détection de visage configuré pour exécuter des algorithmes de détection de visage ;
un module de détection de corps configuré pour calculer des algorithmes de détection de corps ;
un module de détection des repères faciaux configuré pour calculer des algorithmes de détection de repères faciaux ; et
des moyens de traitement configurés pour :
délimiter sur l'au moins une image en vue de dessus une première zone correspondant à une zone du lit où se trouve un oreiller et une deuxième zone correspondant au reste du lit ;
à l'aide du module de détection de visage et du module de détection de corps, générer respectivement des boîtes englobantes de visage et des boîtes englobantes de corps respectives dans l'au moins une image, les boîtes englobantes de visage contenant une partie détectable du visage des personnes et les boîtes englobantes de corps contenant une partie détectable des corps des personnes dans l'au moins une image, dans lequel une partie détectable du visage et une partie détectable du corps comprennent respectivement des parties du visage et des parties du corps que l'algorithme de détection de visage et l'algorithme de détection de corps sont en mesure de détecter parce qu'elles sont visibles ou parce que les algorithmes sont en mesure d'estimer leur présence, leur emplacement et leur contour ;
lorsqu'au moins une boîte englobante de visage a été générée, détecter à l'aide du module de détection des repères faciaux des points de repère clés à l'intérieur de l'au moins une boîte englobante de visage correspondante ;
lorsqu'au moins une boîte englobante de visage a été générée, vérifier une position des points de repère clés détectés et des coins de l'au moins une boîte englobante de visage par rapport à la première zone et à la deuxième zone ;
lorsqu'au moins une boîte de corps a été générée, vérifier la position de l'au moins une boîte englobante de corps par rapport à la première zone ;
déterminer la présence d'une personne alitée ou en repos au lit dans l'au moins une image :
lorsqu'au moins un des points de repère clés est situé à l'intérieur de la première zone ou de la deuxième zone ;
et/ou
lorsqu'au moins un point appartenant à l'au moins une boîte englobante générée est situé à l'intérieur de la première zone ou lorsqu'au moins un point appartenant à la première zone est situé à l'intérieur de l'au moins une boîte englobante de corps générée.

14. Système selon la revendication 13, comprenant un module de détection de points clés de corps configuré pour exécuter des algorithmes de détection de points clés de corps de manière à détecter des points clé à l'intérieur de l'au moins une boîte de corps générée pour estimer une posture corporelle de la personne.

15. Système selon la revendication 14, comprenant un module de classification de postures corporelles configuré pour exécuter des modèles de classification de postures de sorte à déterminer si la personne est allongée sur le sol sur la base de la posture corporelle estimée de la personne.
